# EUROPEAN PATENT SPECIFICATION

(11) **EP 3 522 842 B1**
(45) Date of publication and mention of the grant of the patent: **26.02.2020**
(21) Application number: 17780337.6
(22) Date of filing: 26.09.2017
(51) Int. Cl.: A61F 11/08, B26B 21/40, B26B 19/38, H04R 1/10

(54) **EARTIP, POSITION REFERENCE, AND HAIR PROCESSING SYSTEM**
OHRSTÖPSEL, POSITIONSREFERENZ UND HAARVERARBEITUNGSVORRICHTUNG
EMBOUT, RÉFÉRENCE DE POSITION ET SYSTÈME DE TRAITEMENT DES CHEVEUX

(30) Priority: 06.10.2016 EP 16192562
(43) Date of publication of application: 14.08.2019
(73) Proprietor: Koninklijke Philips N.V., 5656 AE Eindhoven (NL)
(72) Inventor: VAN DEN HOUT, Frans Ruben, 5656 AE Eindhoven (NL); BERNTSEN, Luc, 5656 AE Eindhoven (NL)
(74) Representative: Uittenbroek, Arie Leendert
(86) International application number: PCT/EP2017/074285
(87) International publication number: WO 2018/065255

(56) References cited:
- EP-A2- 1 643 800
- WO-A2-2004/068896
- US-A- 3 258 533
- US-A1- 2012 039 500

## Description

### FIELD OF THE INVENTION

The present disclosure relates to an eartip, particularly a positioning design for an earpiece. Further, the present disclosure relates to a position reference or a position sensor/transducer that is arranged to be attached to a subject's ear, particularly to be placed in the auricle.

In a more specific context, the present disclosure relates to an automated hair processing system. More particularly, the present disclosure relates to a positioning and retaining structure that may be used in the context of automated hairstyle processing and hair cutting systems, particularly for position/orientation detection.

However, the above indicated field shall not be interpreted in a limiting sense. Rather, an eartip in accordance with the present disclosure may also be used in the field(s) of audio technology, noise protection and sound attenuation, for instance.

In specific embodiments, the present disclosure relates to a position monitoring arrangement for an automated hair processing system and to an automated hair processing system that is provided with a position monitoring arrangement.

As used herein, automated hair processing (or: hairstyling) particularly relates to an approach that involves processing, particularly cutting, a subject's hair with an appliance that is capable of automatically adjusting at least one operation parameter, particularly a cutting length, depending on or as a function of an actual location of the processing appliance with respect to the individual subject. Automated hairstyle processing may also be referred to as automatic, semi-automatic or smart hairstyle processing.

The term automated hairstyle processing does not necessarily exclude any human/manual contribution or intervention. For instance, hand-held and hand-guided hair cutting appliances may be used which implement an automated adjustment of an actual cutting length. Hence, automated hairstyling within the context of the present disclosure may also be referred to as computer-aided or computer-assisted smart hairstyling. Computing capacity may be provided by the hair processing appliance itself or by another device that is coupled thereto, such as a mobile phone, a tablet computer, etc.

### BACKGROUND OF THE INVENTION

Haircutting and hairstyling are, to a great extent, manual tasks which typically require a skilled and experienced operator (hair stylist, hair dresser, etc.) who performs a haircut and/or hairstyling operation at a user. Generally, even if the user is satisfied with a particular haircut or hairstyle, the manual task needs to be performed repeatedly, for instance every four to eight weeks for relatively short haircuts. Further, even a well-experienced hairdresser or hairstylist cannot always exactly reproduce a certain haircut. The hairdresser may, on the one hand, imagine the to-be-applied haircut based on the current (grown) state of the hair. On the other hand, the hairdresser may recall and visualize the originally processed state of the previously performed haircut. Further, a user may choose and request a certain haircut by pointing at a visual representation of his/her own or other people wearing a model haircut.

Several attempts have been made to provide smart haircutting appliances which allow a user to cut his/her hair or the hair of another person in a machine supported and controlled fashion. To this end, a haircutting appliance may be provided which is arranged to adjust a present cutting length dependent on a certain position at the head of the to-be-treated person. In other words, the desired haircut is stored in a computing device which is arranged to operate the haircutting appliance accordingly, for instance by adjusting a movable spacing comb. This basically requires that the model of the haircut is already stored in the computing device. It has been proposed to record machine parameters of the haircutting appliance when the haircut is actually performed so that the haircutting procedure can be repeated at a later time.

In this context, WO 2013/163999 A1 discloses a programmable hair trimming system comprising a hair trimming device, said hair trimming system being arranged to detect, by means of an electromagnetic tracking system, the position of the hair trimming device in relation to the head of a person on whom a hair trimming is being performed; relate said position to previously generated hair length profile data regarding the desired hair trimming length at various positions; and automatically and dynamically adjust the hair trimming length of said hair trimming device according to its present position and the hair length profile data.

There is still a certain need for improvements in and alternative approaches to automated haircut/hairstyle processing. In particular, position detection and monitoring still faces major challenges. As the object of the hair processing procedure is typically a human head, it is required to detect the position of the hair processing appliance with reasonable accuracy and to match information describing the intended hair processing operation (e.g. a hair topology model/hair length profile) with the actual head/scalp shape.

Hence, there is still room for improvement in automated haircut recording appliances and methods.

For instance, it has been proposed to use ear-wearable position references/sensors that are arranged to cooperate with a hair processing appliance so as to detect the position/orientation of the appliance with respect to the head or scalp of the treatment subject (whose hair is processed).

Such an ear-wearable component needs to be placed properly and sufficiently fixed as accurate positon detection and orientation detection is crucial for the desired outcome of the hair processing operation. In particular, an in-ear rotation or slipping free from the intended position should be avoided as much as possible.

Similar challenges exist in the field of audio technology (earbuds or earphones), noise protection, sound attenuation, worker safety, noise control, etc.

On the one hand, an ear-wearable device should be arranged to be attached in a reliable, loss-proof or slip-proof fashion. On the other hand, the ear-wearable device shall be designed to be considerably ergonomic and to be worn comfortably and conveniently by a user.

Ear-wearable devices are known that implement soft structures, for instance earplugs made from foam, such as memory foam made from polyvinyl chloride or polyurethane. Further, ear-wearable devices are known that are arranged to be pressed against and to engage specific anatomical features of the ear. Either approach has specific advantages and drawbacks.

In this context, reference is made to EP 1 643 800 A2 that discloses an universal earpiece for securing an acoustic device to an ear of a user, comprising a main body, an auditory canal grommet configured to project into an auditory canal of the user when the earpiece is worn by the user, and a single spring element arranged as a projection from the main body, wherein the main body, the auditory canal grommet and the spring element are sized, configured and arranged such that the earpiece can be accommodated essentially within a concha of the user and be fixed within the concha by applying a rotational movement to the earpiece.

US 3,258,533 A discloses a unitary plastic ear mold adapted to conform to the outer ear and thereby block the transmission of ambient sound to the middle ear, and having a single projection for retaining said mold in the auricle of said outer ear.

### SUMMARY OF THE INVENTION

It is an object of the present disclosure to provide an improved ear-mountable positioning and retaining structure that is arranged to be attached to an ear auricle in an accurate and reliable fashion. Preferably, the structure is arranged to be retained in the mounted state in a considerably slip-free or shift-free fashion. Further, preferably, the positioning and retaining structure is comfortable to wear and easy to attach for a wearer. Preferably, the structure is arranged to be worn by a variety of wearers, having considerably deviating ear shapes, in a robust fashion and substantially insensitive to movements and impacts.

It is a further object of the present disclosure to present beneficial uses for such a positioning and retaining structure in the field of audio technology, noise protection, automated hair processing, etc.

In specific embodiments, it is an object of the present disclosure to provide a position reference for use in an automated hair processing system, the position reference comprising an ear-wearable structure that is easy to attach and that may be placed in an accurately positioning and repeatable fashion.

Further, in specific embodiments, it is an object of the present disclosure to provide a position monitoring arrangement for an automated hair processing system, and an automated hair processing system that address at least some of the above-indicated issues.

In connection with particular embodiments, improvements in position monitoring are sought for. Preferably, embodiments in accordance with the present disclosure enable a robust and accurate position detection and tracking of a hair processing appliance with respect to the head/scalp of the treatment subject (whose hair is processed). Further, it is desired to provide more robust and less error-prone position monitoring approaches that may be utilized in the field of automated hair processing.

In a first aspect of the present disclosure, an eartip is presented that is arranged to be attached to an auricle, the eartip comprising:
- a main body forming a seat portion,
- an extension stub extending from the main body,
- a first arm extending from the main body, and
- a second arm extending from the main body,
- wherein the first arm and the second arm are angularly offset from one another,
- wherein the first arm comprises a free end forming a first arm tip,
- wherein the second arm comprises a free end forming a second arm tip, and
- wherein the first arm and the second arm are arranged to be pressed against the auricle.

This aspect is based on the insight that an eartip may be adequately adapted to a present shape of a (human) auricle in such a way that an accurate and reliable fit may be achieved. As used herein, an eartip within the context of the present disclosure may also be referred to as positioning and retaining piece or positioning and retaining structure.

Whenever reference is made herein to ear anatomy, then the main focus is on an ear of a human user, while this is not intended to be understood in a limiting sense. The ear anatomy of course varies among individuals, but there are characteristics that are somewhat generic among a great percentage of humans.

The first arm, the second arm, and the extension stub form separate projections at the eartip. However, the extension stub is basically integrally shaped with and forms a design feature of the main body. The main body exhibits a compact design which includes the extension stub.

By contrast, the first arm and the second arm are designed to be more distinct as they have a more prominent shape, orientation and extension in relation to the main body. The fist arm and the second arm are basically radially (and to some extent also circumferentially) extending from the main body. By contrast, the extension stub is basically axially extending from the main body, which does not exclude a certain inclination.

The main body may be arranged to be pressed against a central portion of the auricle. The first arm and the second arm may form distinct attachment spots that are spaced from one another and that protrude from the main body. The extension stub forms an extension of the main body that extends towards the ear canal when the eartip is worn. Hence, in certain embodiments, the extension stub is arranged to engage at least an outer portion of the ear canal (auditory canal).

In certain embodiments, the first arm and the second arm are arranged in basically the same plane that is perpendicular to a central axis through the main body. In certain embodiments, the first arm and the second arm extend in a plane that is basically perpendicular to a central axis through the main body.

In certain embodiments, a main extension direction of the first arm and a main extension direction of the second arm are basically parallel to a main extension plane of the auricle, i.e. approximately parallel or tangential to the surface of the head of the wearer in the vicinity of the auricle.

The first arm may also be referred to as rear arm and/or bottom arm. The second arm may also be referred to as front arm and/or top arm. As used herein, the term top refers to a top of the head of the wearer, whereas the term bottom refers to the bottom of the head of the wearer. The term front refers to the front of the head of the wearer. The term rear refers to the back of the head of the wearer.

In an exemplary embodiment, the main body, the first arm and the second arm form a three-point mounting for the eartip of the auricle. Accordingly, the main body defines a first mounting point or mounting region. The first arm defines a second mounting point or mounting region. The second arm defines a third mounting point or mounting region. A three-point mounting for the eartip has the advantage that a determined mounting is possible which reduces the risk of an undesired shift, or slipping off of the eartip.

In an exemplary embodiment of the eartip, the first arm and the second arm are tapered towards their ends, wherein preferably spherical end surfaces are formed at the ends of the first arm and the second arm. Hence, the first arm and the second arm may, respectively, reach and contact a region of the auricle that is considerably distant or spaced from the main body.

In a further exemplary embodiment of the eartip, a first arm and a second arm are elastically deformable. Preferably, in certain embodiments, at least one of the first arm and the second arm is made from an elastic material. More particularly, the first arm and the second arm may be arranged in a resiliently deformable fashion. In accordance with a further exemplary embodiment of the eartip, at least the first arm or the second arm is made from an elastic material. The elastic material may be selected from a group comprising silicone, rubber, synthetic rubber, thermoplastic elastomer material, etc., but not limited thereto.

It is beneficial to design the first arm and the second arm in an elastically deformable fashion. As a result, the eartip may be arranged to fit a variety of wearers involving differently shaped ears and auricles.

In a further exemplary embodiment of the eartip, the main body is arranged to be placed in an auricular cavity. Preferably, the main body is arranged to be pressed against the antitragus. Further, in an exemplary embodiment, the main body is arranged to cover, at least partially, the ear canal. Hence, the main body is arranged to be placed in a central portion of the auricle that adjoins the outer end of the ear canal. The auricular cavity may also be referred to as cavum conchae.

In yet another exemplary embodiment of the eartip, the main body is a plug-shaped main body comprising a seat portion that is arranged to engage the cavum conchae. As used herein, the cavum conchae may be regarded as concha bowl of the auricle.

In accordance with a further exemplary embodiment, the main body, particularly an extension thereof, is arranged to engage, at least partially, the ear canal. In accordance with this embodiment, the main body may comprise an inclined extension stub that protrudes towards the ear canal. In specific embodiments, the main body comprises an inclined extension stub that is arranged to engage the ear canal. Preferably, the extension stub is tapered. A main extension direction of the extension stub is inclined with respect to a main central axis of the main body. A main extension direction of the extension stub is adapted to an assumed orientation of an outer portion of the ear canal.

In yet another exemplary embodiment of the eartip, the first arm is arranged to be pressed against the antihelix. Preferably, in specific embodiments, the first arm is arranged to engage a recess defined by the antihelix. Generally, the antihelix may be regarded as a Y-shaped structure of the auricle that is adjacent to the cavum conchae. Hence, in some specific embodiments, the first arm is arranged to be pressed against the bottom part of the "Y".

In another exemplary embodiment of the eartip, the second arm is arranged to be pressed against the antihelix. Preferably, in specific embodiments, the second arm is arranged to engage a recess defined by the antihelix. In certain embodiments, an engagement region of the second arm is closer to the top of the head of the wearer (i.e. higher) than an engagement region of the first arm. In other words: an engagement region of the second arm may, in a mounted state, be closer to a top region of the auricle than an engagement region of the first arm. This may involve, in certain embodiments, that the first arm is arranged to be pressed against an arm of the branched upper part of the "Y" defined by the antihelix.

It has been observed that in many individuals a rim-like structure is formed, at least sectionally, at the transition between the antihelix and the cavum conchae. This is insofar beneficial as at least one of the first arm and the second arm may be arranged to engage such a recess which is preferably arranged in an undercut fashion. As a result, a certain level of a holding force or retention force may be provided.

In yet another exemplary embodiment of the eartip, the second arm comprises a flexural strength that is greater than the flexural strength of the first arm. In this way, the first arm is more easy to flex when the eartip is attached which has the effect that a better adaption of the eartip to anatomic variations among a variety of wearers is achieved. Further, a rebound force of the second arm, having a greater flexural strength than the first arm, may be greater than a rebound force of the first arm. This may have the effect that a deformation of the second arm has a greater influence on the mounting position of the eartip than a deformation of the first arm. As a result, a main biasing direction in which the main body is urged into a seat at the auricular cavity is present.

A goal of the proposed design of the eartip is to enable an accurate positioning thereof, particularly of the main body. As the first arm is connected to the main body at a side of the main body that is basically opposite to a recess of the cavum conchae which transitions into the outer portion of the ear canal, a rebound force of the first arm and/or the second arm basically pushes the main body into an even firmer and more defined fit at the central portion of the auricle.

In certain embodiments, at least one of the first arm, the second arm and the main body, particularly the inclined extension stub thereof, engages an undercut portion of the ear, when viewed from a lateral side of the head of the wearer.

In a further exemplary embodiment of the eartip, the first arm is made from a material having a Shore durometer in the range of 15 to 40 Shore A, and the second arm is made from a material having a Shore durometer in the range of 70 to 95 Shore A. As used herein, a Shore A durometer value is measured in accordance with ISO 7619-1:2010.

In accordance with another exemplary embodiment, the first arm is made from a material having a Shore durometer that is lower than the Shore durometer of a material the second arm is made from. Again, the Shore A durometer measured in accordance with ISO 7619-1:2010 may be referred to in this context.

Generally, given the Shore durometer scale, a low Shore value represents a basically soft material, whereas a high Shore value represents a basically stiff/hard material.

In yet another exemplary embodiment of the eartip, the first arm is shorter than the second arm and protrudes from a circumferential surface of the main body in a basically tangential direction. The first arm may be at least slightly curved.

In yet another exemplary embodiment of the eartip, the second arm is longer than the first arm. Preferably, the second arm protrudes from a circumferential surface of the main body in a basically radial direction.

In yet another exemplary embodiment of the eartip, in an unbiased state, at least one of the first arm and the second arm are curved, preferably in a frontal or upward direction. This may involve that the tips of the first arm and the second arm are basically pointing towards the face of the head of the wearer.

In yet another exemplary embodiment of the eartip, in an unbiased state, the first arm and the second arm are curved, having the same direction of curvature. Preferably, in specific embodiments, a radius of curvature of the first arm is greater than a radius or curvature of the second arm. In other words, the second arm is having a higher curvature than the first arm. The first arm is having a lower curvature than the second arm. In other words, the first arm is less curved than the second arm.

It is to be noted that the above embodiment does not exclude that in other specific embodiments only one of the first arm and the second arm is curved.

Further, the frontal/upward orientation of at least one of the tips of the first arm and the second arm is present when the eartip is attached to the auricle in an appropriate intended fashion.

In yet another exemplary embodiment, the eartip is a multi-component part. In specific embodiments, the eartip is a multi-component molding part. Accordingly, the eartip is formed from at least two components. Preferably, in certain embodiments, the eartip is formed from and includes at least two components having different material properties. As already indicated above, in certain embodiments, the second arm is more rigid than the first arm. A difference in the flexural strength of the first arm and the second arm may be achieved by design measures, for instance by respectively defining the cross-sectional area and/or the length of the arms. A further potential measure is to form the first arm and the second arm from different materials having different properties.

Hence, through multi-component injection molding, an eartip may be formed which provides load-dependent and purpose-dependent material properties at respective sections, particularly at the first arm and the second arm. Further, forming the multi-component structure in a combined molding process has the advantage that a separate assembly procedure for distinct and separate components may be avoided.

In a further exemplary embodiment of the eartip, a connector interface is provided at the main body that is arranged to be engaged by a mating interface of an attachment part. Hence, in accordance with this embodiment, the eartip may be arranged as a support or a carrier for an attachment part. The attachment part may be for instance arranged as a position reference or a position sensing unit. Further, in alternative embodiments, the attachment part may be an earphone component, e.g. a miniaturized speaker.

The connector interface may be arranged to provide a snap connection between the eartip and the attachment part. At the main body, a cavity for receiving the mating interface may be provided. In some specific embodiments, the attachment part may be entirely or nearly entirely received in the cavity at the main body.

In some specific embodiments of the eartip, a sound channel or sound conduction channel may be formed at and extend through the main body. In case an extension stub is provided that protrudes towards the ear canal, the sound conduction channel may extend also therethrough.

In yet another specific embodiment of the eartip, a visual indicator is provided at the second arm. In this way, a clear indication and insertion aid is provided. Hence, attachment and positioning errors may be reduced or avoided. Further, in case a right side and a left side eartip are provided, also the likelihood of confusion between the two sides may be reduced or even avoided.

In another aspect of the present disclosure there is provided a set of eartips comprising a left side eartip and a right side eartip in accordance with at least one embodiment as discussed herein, wherein the left side eartip and the right side eartip are basically mirror-symmetric. Hence, an appropriate eartip for the left ear and an appropriate eartip for the right ear may be provided.

In yet another aspect of the present disclosure, a position reference for an automated hair processing system is presented, the position reference comprising a reference unit and an ear-mountable eartip in accordance with at least one embodiment as discussed herein, wherein the reference unit is attached to or arranged at the eartip. As already discussed above, the eartip may provide for an accurate and reliable positioning and orientation of the reference unit with respect to the wearer's head. This may significantly enhance the overall position/orientation detection quality and enhance the performance of the hair processing system.

In certain embodiments, the reference unit may be arranged to provide audio feedback or sound feedback. Hence, a miniaturized loudspeaker may be arranged at the reference unit. Through an appropriate sound conduction channel, sound may be transmitted to the ear canal of the user.

The reference unit may be arranged as an active reference unit and/or a passive reference unit. An active reference unit may be also referred to as position sensor, position transducer, etc.

In yet another aspect of the present disclosure, an automated hair processing system, particularly a haircutting system, is presented, the system comprising a portable hand-held hair processing appliance, a hair processing unit arranged at the appliance, and a position monitoring arrangement comprising at least one position reference in accordance with at least one embodiment as described herein, wherein at least one feature of the hair processing appliance is controllable dependent on at least one of an actual orientation and an actual position of the hair processing appliance with respect to a head of a treatment subject.

As used herein, the treatment subject may be regarded as the person whose hair is processed. Hence, the treatment subject may be a passive user, i.e. an operator of the appliance and the treatment subject are not the same person. In the alternative, the treatment subject may be an active user, i.e. the operator of the appliance and the treatment subject are the same person.

Further, in certain specific aspects, an eartip in accordance with at least one embodiment as discussed herein may be used for producing/assembling a position reference for an automated hair processing system that is arranged to be attached to an ear of a treatment subject. Further, the eartip may be used for producing/assembling an in-ear monitor or an earphone. In an alternative embodiment, an eartip in accordance with at least one embodiment as discussed herein may be used for producing/assembling a noise protection ear plug or a sound attenuation ear plug.

As discussed above, the eartip may be used in different fields of application as the benefits provided due to the novel features discussed herein become apparent in different applications of ear-wearable devices.

Preferred embodiments of the disclosure are defined in the dependent claims. It shall be understood that the claimed method has similar and/or identical preferred embodiments as the claimed device and as defined in the dependent claims.

### BRIEF DESCRIPTION OF THE DRAWINGS

These and other aspects of the disclosure will be apparent from and elucidated with reference to the embodiments described hereinafter. In the following drawings
Fig. 1 shows a simplified general layout of an exemplary embodiment of an automated hair processing system;
Fig. 2 shows a simplified schematic view of a (right) ear auricle wherein the view orientation corresponds to a partial lateral view of a treatment subject's or wearer's head;
Fig. 3 shows a perspective frontal top view of an embodiment of an eartip in accordance with the present disclosure;
Fig. 4 shows a perspective top view of an eartip in accordance with the arrangement of Fig. 3, wherein the view orientation of Fig. 4 is different from the view orientation of Fig. 3;
Fig. 5 shows a top view of an eartip in accordance with Fig. 3 and Fig. 4;
Fig. 6 shows a lateral view of the eartip of Fig. 5;
Fig. 7 shows an opposite lateral view of the eartip of Fig. 6;
Fig. 8 shows a bottom view of the eartip illustrated in Figs. 5, 6 and 7;
Fig. 9 shows a simplified combination of Fig. 2 and Fig. 6, wherein the eartip shown in Fig. 6 is arranged in Fig. 9 in an intended mounting position at an auricle but in an unbiased/relaxed state of the first arm and the second arm;
Fig. 10 is a simplified view of an eartip in a mounted position in an auricle, wherein the first arm and the second arm are shown in a deflected, biased state;
Fig. 11 is a perspective exploded view of a position reference unit comprising an eartip and an attachment part;
Fig. 12 is a lateral view of the attachment part of Fig. 11; and
Fig. 13 is a side view of the attachment part shown in Fig. 11 and Fig. 12.

### DETAILED DESCRIPTION OF THE INVENTION

In the following, several embodiments and features of eartips in accordance with the present disclosure will be discussed and described in more detail. Primarily for illustrative purposes, reference is made to a certain field of application, namely to automated hairstyle processing systems in which a position detection arrangement can make use of a more accurate and reliable mounting of ear-wearable devices. However, the exemplary field of application shall not be construed in a limiting sense. As discussed above, further fields of application for the eartips in accordance with the present disclosure may be envisaged.

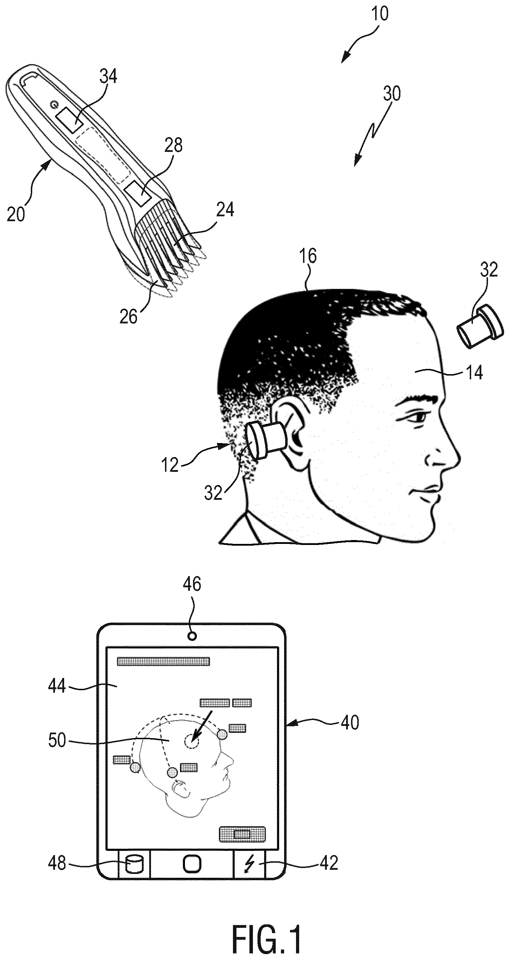

Fig. 1 shows a simplified schematic illustration of an automated hair processing system 10 which may be also referred to as automated hairstyle processing system. The system 10 may be also referred to as automated haircut processing system. In specific embodiments, as already indicated further above, the present disclosure generally relates to grooming, processing and/or styling human hair and animal hair which may involve head hair and body hair.

The system 10 is arranged to perform a haircut or hairstyle operation at a client or subject 12 in a computer-aided automatic and/or semi-automatic fashion. A field of application for the system 10 may be a hair processing operation applied to the user itself. Hence, in the following the subject 12 will be referred to as user, even though a third person may operate the appliance. Needless to say, also another person may operate the system 10.

In Fig. 1, a haired portion 16 at a head or scalp portion 14 of the user 12 is illustrated. Generally, the system 10 is arranged as a "smart" haircutting or hairstyling system.

The system 10 comprises a haircutting appliance 20 which may also be referred to as automated haircutting appliance. The appliance 20 is arranged as a hand-held or hand-guided appliance. Generally, the appliance 20 may be similar to a standard non-automated haircutting appliance but may be augmented and supplemented by additional processing and control features. Generally, the device 20, particularly a housing 22 thereof, may be grasped by a user and operated so as to cut hair. This may involve moving the device 20 through the haired portion 16 at the head portion 14 of the user 12 and cutting hairs to a desired length. The device 20 may be held and operated by the subject 12 itself (whose haircut is to be processed). In the alternative, the device 20 may be operated by another individual.

The device 20 comprises a processing unit 24 which is attached to the housing 22. The processing unit may involve, for instance, a blade set. Further, a comb 26 may be coupled with the processing unit 24. In at least some embodiments, the comb 26 is arranged as an adjustable spacing comb. Further, an adjustment unit 28 for the comb 26 is provided. Generally, the comb 26 defines an offset between a skin or scalp level at the subject 12 and the processing unit 24. Consequently, the hair length may be controlled.

In automated hair processing systems 10, the operation of the appliance 20 may be controlled dependent on an actual position of the appliance 20 with respect to the user 12. To this end, a position monitoring arrangement 30 is provided. The position monitoring arrangement 30 comprises a plurality of position references 32. For instance, as shown in Fig. 1, two position references 32 may be provided. Each of the position references 32 shown in the exemplary embodiment illustrated in Fig. 1 is arranged as an ear-wearable or ear-mountable position reference 32, using an eartip. More particularly, at least in some embodiments, the position reference 32 may be referred to as ear plug reference or ear bud reference. In some exemplary embodiments, at least one of the position references 32 may be arranged as position sensor or position transducer.

The position references 32 may be arranged as active component or passive component. An active component may be arranged as a component that is capable of communicating with another device, e.g. with a transmitter. For instance, an active component may emit reference signals that make it possible to sense an absolute or relative position and/or orientation between involved components. A passive component may be arranged as a component that is not capable of communicating with another device but that is primarily sensed by another (active) device.

Further, a transmitter 34 may form part of the position monitoring arrangement. In the arrangement as shown in Fig. 1, the transmitter 34 is implemented in the appliance 20. The transmitter 34 cooperates with the position references 32. As a result, a position and orientation of the appliance 20 with respect to the head 14 of the user 12 may be detected and tracked. In regard of a general layout of a position detection system for an automated hair processing system, reference is made again to WO 2013/163999 A1.

In general, the main purpose of the position monitoring arrangement 30 is to detect a current position of the appliance 20, particularly of the processing unit 24 thereof, with respect to the haired portion 16 of the head portion (scalp) 14 of the subject 12. Consequently, the actual position of the appliance 20 may be assigned to a respective hair property value, particularly to a hair length value. As a result, automated hair processing is enabled as the adjustment unit 28 may be automatically operated so as to adjust the comb 26 accordingly.

As further shown in Fig. 1, also a computing device 40 may form part of the system 10. This may be for instance the case when the appliance 20 as such does not provide sufficient data processing and computing capacity. By way of example, the computing device 40 may be arranged as a mobile device such as a tablet computer, a mobile phone, etc. The computing device 40 comprises a processing unit which implements at least one controller 42. The controller 42 may be also referred to as position controller.

Further, user feedback units 44, 46 may be provided as to establish an interaction between the user and the appliance 20 via the computing device 40. For instance, user feedback may be provided via a display 44 and via speakers 46. Further, a memory unit 48 may be arranged at the computing device 40. The memory unit 48 may be used to store hairstyle and/or haircut models. More generally, operational data may be stored in the memory unit 48. In Fig. 1, visual information 50 is displayed on the screen 44. Hence, visual operator guidance may be provided which further facilitates the hair processing operation.

A head topology model and a hairstyle model may be stored in the memory unit 48. Hence, a respective hair length value may be assigned to a specific scalp portion.

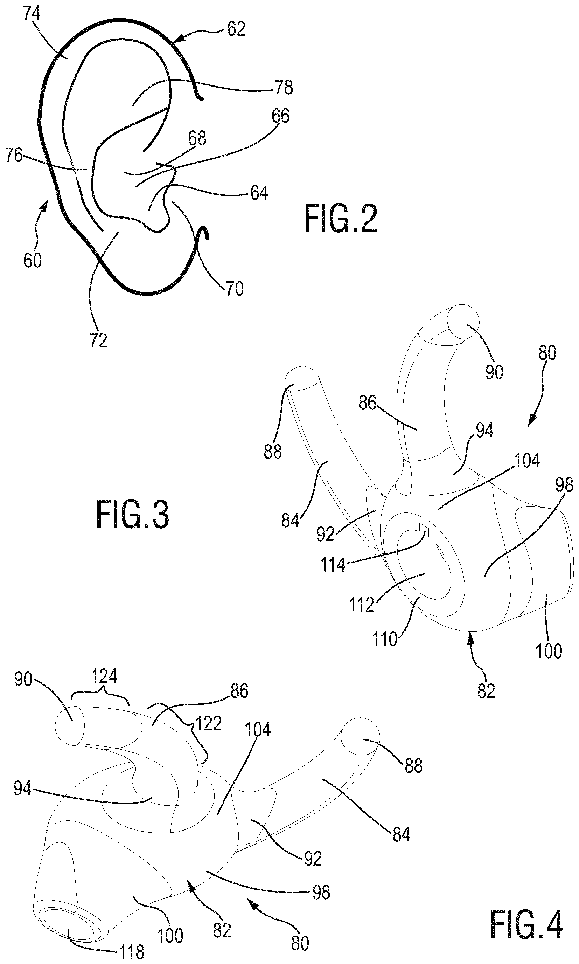

Fig. 2 is a schematic simplified frontal view of an ear 60. The view orientation of Fig. 2 corresponds to a lateral view of a head of a treatment subject for the automated hair processing system 10 illustrated in Fig. 1. Fig. 2 shows a right-side ear. Needless to say, a left side ear would be arranged in a basically mirror-symmetric fashion.

The ear 60 comprises an auricle 62 which may be regarded as the part of the ear 60 that is arranged outside of the head. The auricle 62 adjoins an ear canal 64 which extends into the head. Adjacent to the ear canal 64, an auricular cavity 66 is provided which may generally be referred to as concha or cavum conchae 68.

Further characteristic portions of the auricle 62 are the tragus 70 and the antitragus 72. The antitragus 72 is opposite to the tragus 70. Between the tragus 70 and the antitragus 72, the ear canal 64 transitions into the cavum conchae 68.

At an edge region of the auricle 62, the helix 74 is provided. Between the helix 74 and the cavum conchae 68, the antihelix 76, 78 is provided. Reference numeral 76 indicates a first, bottom part of the antihelix. Reference numeral 78 indicates a second, top part of the antihelix 78. Generally, the antihelix may be arranged in a Y-shaped fashion wherein reference numeral 76 indicates the bottom leg of the "Y", and wherein reference numeral 76 indicates one of the branched arms of the "Y".

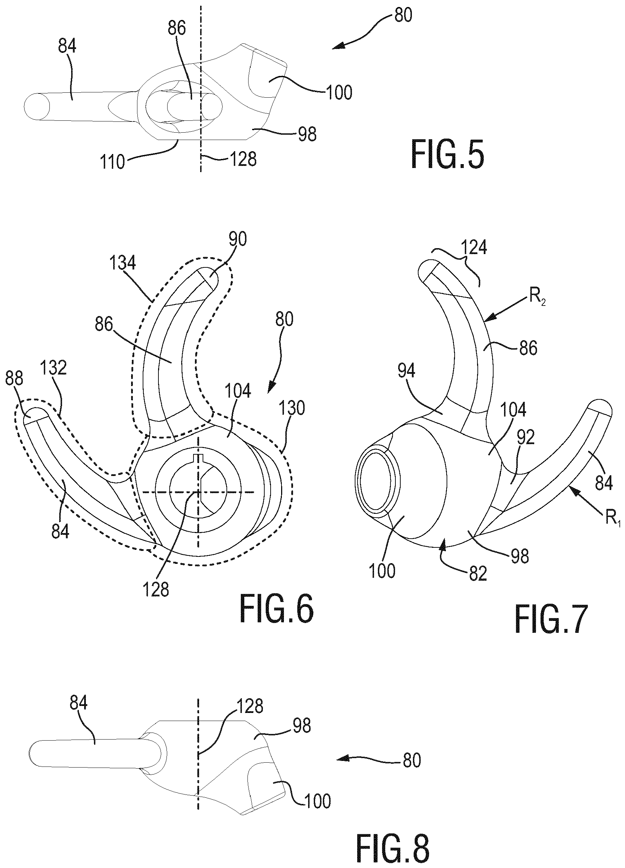

With particular reference to Fig. 3 and Fig. 4, and with additional reference to Figs. 5 to 8, an exemplary embodiment of an eartip 80 will be described and discussed in more detail. As used herein, position and orientation indications generally refer to the head of the wearer of the eartip 80. Hence, a top side corresponds to the top of the head. A bottom side corresponds to the bottom of the head and/or the neck. The term front relates to the facial side. The term rear relates to the back of the head. The term lateral relates to the sides of the head where the ears are present.

The eartip 80 is shown in Fig. 3 in a perspective frontal top view and in Fig. 4 in a different perspective view orientation. As shown in Fig. 3 and Fig. 4, the eartip 80 comprises a main body 82. The main body 82 may be arranged in a basically disc-, donut-(torus-) shape and/or ellipsoid form. A first arm 84 extends from the main body 82. Further, a second arm 86 extends from the main body 82. As can be best seen in Fig. 6 and Fig. 7, the first arm 84 may also be referred to as rear arm. The second arm 86 may also be referred to as front or frontal arm. Further, the first arm 84 may be referred to as bottom arm. The second arm 86 may be referred to as top arm. The first arm 84 and the second arm 86 are angularly offset from one another.

Further, as can be best seen from Fig. 5 and Fig. 8, the first arm 84 and the second arm 86 respectively may have a main extension direction which is basically in the same plane. In specific embodiments, the common plane of extension of the first arm 84 and the second arm 86 is perpendicular to a main central axis through the main body 82, refer also to reference numeral 128 in Fig. 5, Fig. 6 and Fig. 8.

At the first arm 84, a first arm tip 88 is formed. At the second arm 86, a second arm tip 90 is formed. The first tip 88 and the second tip 90 respectively have a spherical end surface. Further, the first arm 84 and the second arm 86 are at least slightly tapered along their main extension direction towards the tips 88, 90.

Between the first arm 84 and the main body 82, a first arm connector portion 92 is formed. Between the second arm 86 and the main body 82, a second arm connector portion 94 is formed. Between the main body 82 and the connector portions 92, 94, respectively, a smooth transition or tangential transition is formed.

The main body 82 forms a seat portion 98 of the eartip 80. The seat portion 98 is arranged to be placed against a portion of the auricular cavity 66. More particularly, the seat portion 98 of the eartip 80 may be at least slightly pressed against a rear (inner) side of the antitragus 72 and/or the tragus 70 in the vicinity of the outer end of the ear canal 64.

Further, an inclined extension stub 100 extends or protrudes from the main body 82. An orientation of the extension stub 100 can be best seen in Fig. 5 and Fig. 8. A main extension direction of the extension stub 100 is at least slightly inclined with respect to the main central axis 128 of the main body 82. The position, orientation and extension of the inclined extension stub 100 are adapted to the shape of the ear canal 64 of a variety of wearers. The extension stub 100 may cover the ear canal 64, at least partially. Further, the inclined extension stub 100 may at least slightly extend in and engage the outer portion of the ear canal 64.

Further, a circumferential surface of the donut-shaped or ellipsoid main body 82 is indicated by reference numeral 104. At the outer radial circumferential surface 104, the first arm 84 and the second arm 86 extend from the main body 82.

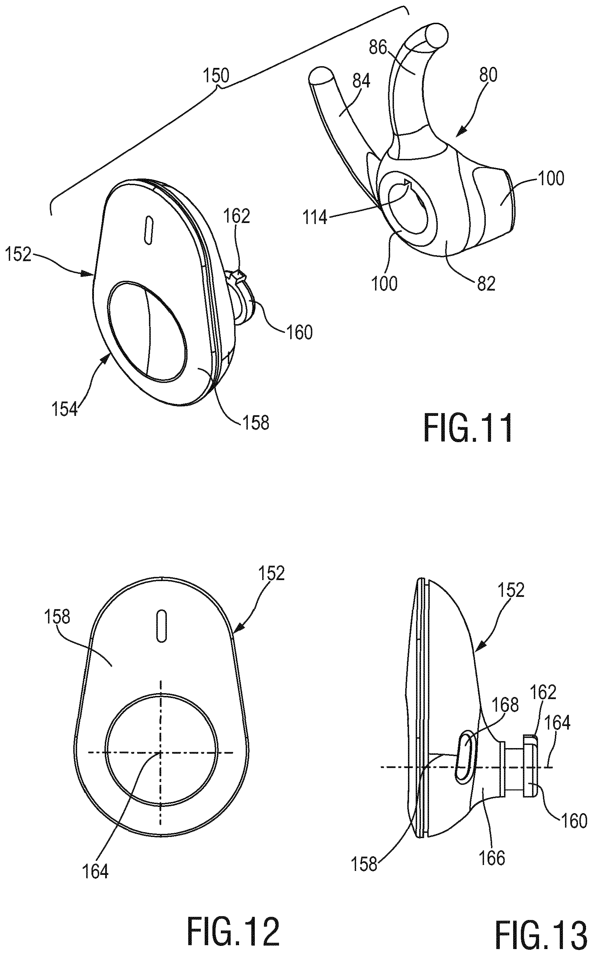

As can be best seen from Fig. 3, a connector interface 110 is formed at an outer, lateral side of the eartip 80. In accordance with the exemplary embodiment shown in Fig. 3, the connector interface 110 comprises a ring-like end surface adjoined by a receiving cavity 112 in the interior of the main body 82. Further, mounting features 114 may be provided. For instance, a snap-lock mounting or a bayonet-mounting may be provided. Hence, the eartip 80 may be used as a carrier or a mounting aid for an attachment part, refer also to Fig. 11 which will be discussed further below.

In some embodiments, a passage 118 is provided which extends through the main body 82 and, if any, the extension stub 100. The passage 118 may also be referred to as sound passage.

As indicated in Fig. 4 by brackets denoted by reference numerals 122 and 124, respectively, a non-highlighted portion 122 and an indicator portion 124 may be present at the second arm 86. The indicator portion 124 may be visually highlighted, for instance by a signal color or a color having a high contrast with respect to a color of the non-highlighted portion 122.

As already indicated above, a central axis 128 through the main body 82 is indicated in Fig. 5, Fig. 6 and Fig. 8. Fig. 6 is a lateral side view of the eartip 80 in an assembly orientation that is aligned with a general orientation of an ear or head of the wearing person. It can be seen that the first arm 86 is slightly curved towards the top side. Hence, the tip 88 of the first arm 84 points towards the top side. Further, it can be seen that the arm 86 is at least slightly curved towards the front side. Consequently, the tip 90 of the second arm 86 points towards the front side. Radii of curvature for the first arm 84 and the second arm 86 are indicated in Fig. 7 by R₁ for the first arm 84 and R₂ for the second arm 86. The radius R₁ of the first arm 84 is larger than the radius R₂ for the second arm 86. Hence, a level of curvature of the second arm 86 is greater than a level of curvature of the first arm 84.

Further, an effective overall extension length of the second arm 86 is slightly larger than an effective overall extension length of the first arm 84. The first arm 84 and the second arm 86 are at least slightly tapered along their main extension. Hence, a cross-sectional profile of the arms 84, 86 at their connector portions 92, 94 is larger than a cross-sectional profile of the arms 84, 86 at or adjacent to their tips 88, 90.

As discussed above, in certain embodiments, the eartip 80 is a multi-component part, preferably a multi-component injection molding part formed from relatively soft elastic material. In Fig. 6, dashed lines 130, 132, 134 indicate respective segments or components. For instance, a base component 130, a first arm component 132 and a second arm component 134 may be provided. Hence, a three-component part may be provided. In some exemplary embodiments, a two-component part may be provided wherein one of the first arm component 132 and the second arm component 134 is integrally (simultaneously) molded with the base component 130.

A goal of the multi-component manufacturing approach is that the first arm 84 and the second arm 86 may be formed from different materials having different properties. In at least some exemplary embodiments, the first arm 84 has a lower stiffness (lower Shore durometer) than the second arm 86. By way of example, the first arm 84 may be made from a material having a Shore A durometer in the range of about 15 to 50 Shore A. By contrast, the second arm 86 may be made from a material having a Shore A durometer in the range of about 70 to 95 Shore A. Hence, a different level of resistance against deformation is present at the first arm 84 and the second arm 86 which may be used to facilitate and improve attachment and positioning of the eartip 80 in an even more accurate fashion.

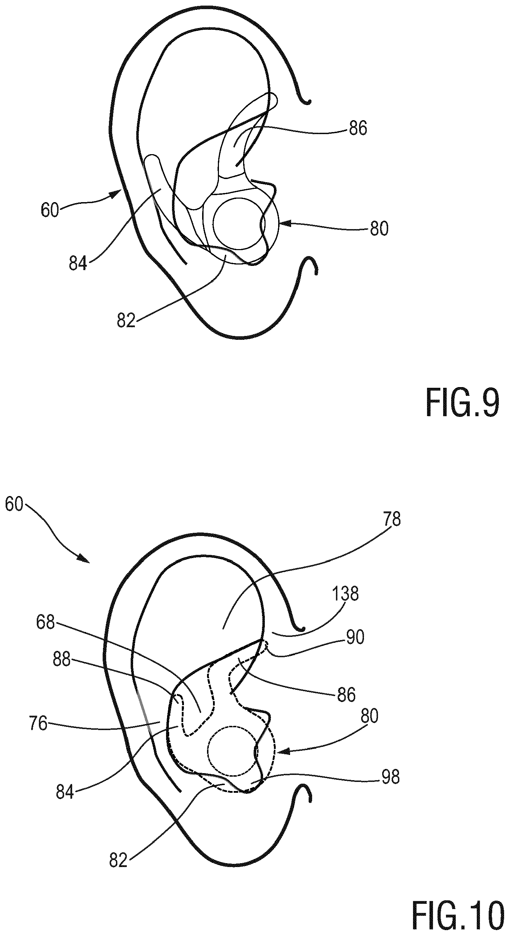

Further reference is made to Fig. 9 and Fig. 10, illustrating an attached state of the eartip 80 at the ear 60. Fig. 9 shows an illustrative overlay of an ear 60 shape as illustrated in Fig. 2 and a respective orientation of the eartip 80 as shown in Fig. 6. In Fig. 9, neither the first arm 84 nor the second arm 86 are shown in a deformed (biased and/or engaged) state.

Fig. 10 illustrates a deformed and mounted state of the eartip 80 at the auricle 62 of the ear 60, wherein both the first arm 84 and the second arm 86 are at least slightly deformed. The first arm 84 is at least slightly deformed in a forward direction, i.e. a tip 88 is in a more frontal position than in an unbiased state. Similarly, also the second arm 86 is at least lightly deformed in a frontal direction, i.e. the tip 90 of the second arm 86 is in a more frontal position than in the unbiased state.

Also the main body 82 is arranged to be pressed against a certain defined portion of the auricular cavity 66. Both the first arm 84 and the second arm 86 may provide a certain rebound force, due to their deformation. As a consequence, the main body 82, particularly the seat portion 98 thereof, is pressed against the auricular cavity 66 in a portion thereof that is adjacent to the tragus 70 and the antitragus 72, refer also to Fig. 2.

It is preferred that at least one of the main body 82, particularly the seat portion 98 thereof, the first arm 84 and the second arm 86 is arranged to engage an undercut portion of the auricle 62. In Fig. 10, an exemplary undercut portion is indicated by reference numeral 138. Hence, the tip 90 of the second arm 86 may engage the undercut portion 138 which may be provided at a rim section defined by the helix 74 and/or the antihelix 78.

As already discussed, a great variety regarding the shape and size of the auricle 62 among different individuals is present. Hence, each of the first arm 84, the second arm 86 and the main body 82, particularly the seat portion 98 thereof, is arranged to engage a respective undercut section, if any. As a result, the eartip 80 is arranged to adequately fit a large share of individuals. However, in certain embodiments, a defined number of differently shaped and/or sized eartips 80 is provided so as to further increase the fraction of individuals in which an eartip 80 in accordance with the present disclosure may be reliably and firmly attached to the ears.

Further reference is made to Fig. 11, Fig. 12 and Fig. 13. Fig. 11 shows a perspective exploded view of a position reference 150 that incorporates an eartip 80 as discussed hereinbefore. It is again noted that the position reference 150 stands for a variety of potential applications for the eartip 80. Hence, the assembly shown in Fig. 11 in an exploded state is not limited to position references for automated hair processing systems.

In accordance with the exemplary embodiment of Fig. 11, the position reference 150 includes an attachment part 152 arranged as a reference unit 154. The attachment part 152 may also be provided for purposes other than position reference/position detection.

In the position reference application, the reference unit 154 may be provided with a coil arrangement that interacts with an electromagnetic field created by a transmitter at a remote appliance. As discussed above, the reference unit 154 may also be arranged as an (active) sensing unit. For ease of reference, the reference unit 154 will be referred to hereinafter as attachment part 152.

Fig. 12 shows a lateral (side) view of the attachment part 152. Fig. 13 shows a corresponding frontal view, wherein the view orientations and designations of Fig. 12 and Fig. 13 are aligned with overall orientations that relate to the head of the wearer of the eartip 80 to which the attachment part 152 may be attached.

The attachment part 152 comprises a housing 158 in which sensors, loudspeakers, batteries, indicators and even controls may be arranged.

As can be best seen in Fig. 11 and Fig. 13, a mating interface 160 is formed at the attachment part 152 which is arranged to cooperate with the connector interface 110 at the main body 82 of the eartip 80. By way of example, the mating interface 160 may comprise a mounting feature 162 that is adapted to the mounting feature 114 of the connector interface 110, refer to Fig. 3. A central axis 164 is formed at the attachment part 152 and extends through a stud 166 that forms part of the mating interface 160. The stud 166 is arranged to engage the receiving cavity 112 of the eartip 80. The mounting feature 162 and the mounting feature 114 may cooperate to provide a snap-on mounting feature or a bayonet-mounting feature.

Further, in certain embodiments, at least one opening 168, particularly a sound opening, may be formed in the housing 158.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. An eartip (80) arranged to be attached to an auricle (62), the eartip (80) comprising:
- a main body (82) forming a seat portion (98),
- an extension stub (100) extending from the main body (82),
- a first arm (84) extending from the main body (82), and
- a second arm (86) extending from the main body (82),
wherein the first arm (84) and the second arm (86) are angularly offset from one another,
wherein the first arm (84) comprises a free end forming a first arm tip (88),
wherein the second arm (86) comprises a free end forming a second arm tip (90), and
wherein the first arm (84) and the second arm (86) are arranged to be pressed against the auricle (62).

2. The eartip (80) as claimed in claim 1, wherein the main body (82), the first arm (84) and the second arm (86) form a three-point mounting for the eartip (80) at the auricle (62).

3. The eartip (80) as claimed in claim 1 or 2, wherein the first arm (84) and the second arm (86) are elastically deformable, and wherein, preferably, at least one of the first arm (84) and the second arm (86) is made from an elastic material.

4. The eartip (80) as claimed in any of the preceding claims, wherein the main body (82) is arranged to be placed in an auricular cavity (66) and, preferably, arranged to be pressed against the against the antitragus (72).

5. The eartip (80) as claimed in any of the preceding claims, wherein the first arm (84) is arranged to be pressed against the antihelix (76), preferably to engage a recess defined by the antihelix (76).

6. The eartip (80) as claimed in any of the preceding claims, wherein the first arm (84) is arranged to be pressed against the antihelix (76), preferably to engage a recess defined by the antihelix (76), and wherein an engagement region of the second arm (86) is, in a mounted state, closer to a top region of the auricle (62) than an engagement region of the first arm (84).

7. The eartip (80) as claimed in any of the preceding claims, wherein the second arm (86) comprises a flexural strength that is greater than the flexural strength of the first arm (84).

8. The eartip (80) as claimed in any of the preceding claims, wherein the first arm (84) is made from a material having a Shore durometer in the range of 15 to 40 Shore A, and wherein the second arm (86) is made from a material having a Shore durometer in the range of 70 to 95 Shore A.

9. The eartip (80) as claimed in any of the preceding claims, wherein the first arm (84) is shorter than the second arm (86) and protrudes from a circumferential surface (104) of the main body (82) in a basically tangential direction.

10. The eartip (80) as claimed in any of the preceding claims, wherein the second arm (86) is longer than the first arm (84), and wherein the second arm (86) protrudes from a circumferential surface (104) of the main body (82) in a basically radial direction.

11. The eartip (80) as claimed in any of the preceding claims, wherein, in an unbiased state, the first arm (84) and the second arm (86) are curved, having the same direction of curvature, and wherein a radius of curvature of the first arm (84) is greater than a radius of curvature of the second arm (86).

12. The eartip (80) as claimed in any of the preceding claims, wherein the eartip (80) is a multi-component injection molding part including at least two components having different material properties.

13. The eartip (80) as claimed in any of the preceding claims, wherein at the main body (82) a connector interface (110) is provided that is arranged to be engaged by a mating interface (160) of an attachment part (152).

14. A position reference (150) for an automated hair processing system (10), the position reference (150) comprising a reference unit (154) and an ear-mountable eartip (80) as claimed in any of the preceding claims, wherein the reference unit (154) is attached to the eartip (80).

15. An automated hair processing system (10), particularly a hair cutting system, the system comprising a portable hand-held hair processing appliance (20), a hair processing unit arranged (24) at the appliance (20), and a position monitoring arrangement (30) comprising at least one position reference (150) as claimed in claim 14, wherein at least one feature of the hair processing appliance (20) is controllable dependent on at least on of an actual orientation and an actual position of the hair processing appliance (20) with respect to a head of a treatment subject.

## Patentansprüche

1. Ohrstöpsel (80), der ausgelegt ist, an einer Ohrmuschel (62) befestigt zu werden, wobei der Ohrstöpsel (80) umfasst:
- einen Hauptkörper (82), der einen Sitzabschnitt (98) bildet,
- einen Verlängerungsstutzen (100), der sich vom Hauptkörper (82) erstreckt,
- einen ersten Arm (84), der sich vom Hauptkörper (82) erstreckt, und
- einen zweiten Arm (86), der sich vom Hauptkörper (82) erstreckt,
wobei der erste Arm (84) und der zweite Arm (86) winkelversetzt zueinander sind,
wobei der erste Arm (84) ein freies Ende umfasst, das eine erste Armspitze (88) bildet,
wobei der zweite Arm (86) ein freies Ende umfasst, das eine zweite Armspitze (90) bildet, und
wobei der erste Arm (84) und der zweite Arm (86) ausgelegt sind, gegen die Ohrmuschel (62) gedrückt zu werden.

2. Ohrstöpsel (80) nach Anspruch 1, wobei der Hauptkörper (82), der erste Arm (84) und der zweite Arm (86) eine Dreipunktbefestigung für den Ohrstöpsel (80) an der Ohrmuschel (62) bilden.

3. Ohrstöpsel (80) nach Anspruch 1 oder 2, wobei der erste Arm (84) und der zweite Arm (86) elastisch verformbar sind und wobei vorzugsweise mindestens einer des ersten Arms (84) und des zweiten Arms (86) aus einem elastischen Material besteht.

4. Ohrstöpsel (80) nach einem der vorstehenden Ansprüche, wobei der Hauptkörper (82) ausgelegt ist, in einer Ohrmuschelhöhle (66) angeordnet zu werden, und vorzugsweise ausgelegt ist, gegen den Antitragus (72) gedrückt zu werden.

5. Ohrstöpsel (80) nach einem der vorstehenden Ansprüche, wobei der erste Arm (84) ausgelegt ist, gegen die Antihelix (76) gedrückt zu werden, vorzugsweise in eine durch die Antihelix (76) definierte Aussparung einzugreifen.

6. Ohrstöpsel (80) nach einem der vorstehenden Ansprüche, wobei der erste Arm (84) ausgelegt ist, gegen die Antihelix (76) gedrückt zu werden, vorzugsweise in eine durch die Antihelix (76) definierte Aussparung einzugreifen, und wobei sich ein Eingriffsbereich des zweiten Arms (86), in einem montierten Zustand, näher an einem oberen Bereich der Ohrmuschel (62) als ein Eingriffsbereich des ersten Arms (84) befindet.

7. Ohrstöpsel (80) nach einem der vorstehenden Ansprüche, wobei der zweite Arm (86) eine Biegefestigkeit umfasst, die größer ist als die Biegefestigkeit des ersten Arms (84) ist.

8. Ohrstöpsel (80) nach einem der vorstehenden Ansprüche, wobei der erste Arm (84) aus einem Material mit einem Shore-Durometer im Bereich von 15 bis 40 Shore A hergestellt ist und wobei der zweite Arm (86) aus einem Material mit einem Shore-Durometer im Bereich von 70 bis 95 Shore A hergestellt ist.

9. Ohrstöpsel (80) nach einem der vorstehenden Ansprüche, wobei der erste Arm (84) kürzer als der zweite Arm (86) ist und von einer Umfangsoberfläche (104) des Hauptkörpers (82) in einer im Wesentlichen tangentialen Richtung vorsteht.

10. Ohrstöpsel (80) nach einem der vorstehenden Ansprüche, wobei der zweite Arm (86) länger als der erste Arm (84) ist und wobei der zweite Arm (86) von einer Umfangsoberfläche (104) des Hauptkörpers (82) in einer im Wesentlichen radialen Richtung vorsteht.

11. Ohrstöpsel (80) nach einem der vorstehenden Ansprüche, wobei in einem nicht vorgespannten Zustand der erste Arm (84) und der zweite Arm (86) gekrümmt sind, die gleiche Krümmungsrichtung aufweisen und wobei ein Krümmungsradius des ersten Arms (84) größer als ein Krümmungsradius des zweiten Arms (86) ist.

12. Ohrstöpsel (80) nach einem der vorstehenden Ansprüche, wobei der Ohrstöpsel (80) ein Mehrkomponentenspritzgussteil ist, das mindestens zwei Komponenten mit unterschiedlichen Materialeigenschaften einschließt.

13. Ohrstöpsel (80) nach einem der vorstehenden Ansprüche, wobei am Hauptkörper (82) eine Verbinderschnittstelle (110) bereitgestellt ist, in die eine Gegenschnittstelle (160) eines Befestigungsteils (152) eingreifen kann.

14. Positionsreferenz (150) für ein automatisiertes Haarverarbeitungssystem (10), wobei die Positionsreferenz (150) eine Referenzeinheit (154) und einen am Ohr anbringbaren Ohrstöpsel (80) nach einem der vorstehenden Ansprüche umfasst, wobei die Referenzeinheit (154) am Ohrstöpsel (80) angebracht ist.

15. Automatisiertes Haarverarbeitungssystem (10), insbesondere Haarschneidsystem, wobei das System ein tragbares Haarverarbeitungsgerät (20), eine am Gerät (20) angeordnete Haarverarbeitungseinheit (24) und eine Positionsüberwachungsanordnung (30) umfasst, die mindestens eine Positionsreferenz (150) nach Anspruch 14 umfasst, wobei mindestens ein Merkmal des Haarverarbeitungsgeräts (20) in Abhängigkeit von mindestens einer tatsächlichen Ausrichtung und einer tatsächlichen Position des Haarverarbeitungsgeräts (20) in Bezug auf einen Kopf eines Behandlungssubjekts ist.

## Revendications

1. Embout d'oreille (80) agencé pour être fixé à une auricule (62), l'embout d'oreille (80) comprenant :
- un corps principal (82) formant une partie de siège (98),
- un talon d'extension (100) s'étendant du corps principal (82),
- un premier bras (84) s'étendant du corps principal (82) et
- un second bras (86) s'étendant du corps principal (82),
dans lequel le premier bras (84) et le second bras (86) présentent un décalage angulaire mutuel,
dans lequel le premier bras (84) comprend une extrémité libre formant un premier embout de bras (88),
dans lequel le second bras (86) comprend une extrémité libre formant un second embout de bras (90) et
dans lequel le premier bras (84) et le second bras (86) sont agencés pour être pressés contre l'auricule (62).

2. Embout d'oreille (80) selon la revendication 1, dans lequel le corps principal (82), le premier bras (84) et le second bras (86) forment un montage en trois points pour l'embout d'oreille (80) sur l'auricule (62).

3. Embout d'oreille (80) selon la revendication 1 ou 2, dans lequel le premier bras (84) et le second bras (86) sont déformables de manière élastique et dans lequel, de préférence, au moins l'un du premier bras (84) et du second bras (86) est constitué d'un matériau élastique.

4. Embout d'oreille (80) selon l'une quelconque des revendications précédentes, dans lequel le corps principal (82) est agencé pour être placé dans une cavité auriculaire (66) et agencé de préférence pour être pressé contre l'antitragus (72).

5. Embout d'oreille (80) selon l'une quelconque des revendications précédentes, dans lequel le premier bras (84) est agencé pour être pressé contre l'anthélix (76), de préférence pour s'engager dans un évidement défini par l'anthélix (76).

6. Embout d'oreille (80) selon l'une quelconque des revendications précédentes, dans lequel le premier bras (84) est agencé pour être pressé contre l'anthélix (76), de préférence pour s'engager dans un évidement défini par l'anthélix (76), et dans lequel une région d'engagement du second bras (86) est, à l'état monté, plus proche d'une région supérieure de l'auricule (62) qu'une région d'engagement du premier bras (84).

7. Embout d'oreille (80) selon l'une quelconque des revendications précédentes, dans lequel le second bras (86) comprend une résistance à la flexion qui est supérieure à la résistance à la flexion du premier bras (84).

8. Embout d'oreille (80) selon l'une quelconque des revendications précédentes, dans lequel le premier bras (84) est constitué d'un matériau ayant un duromètre Shore dans la plage de 15 à 40 Shore A et dans lequel le second bras (86) est constitué d'un matériau ayant un duromètre Shore dans la plage de 70 à 95 Shore A.

9. Embout d'oreille (80) selon l'une quelconque des revendications précédentes, dans lequel le premier bras (84) est plus court que le second bras (86) et fait saillie d'une surface circonférentielle (104) du corps principal (82) dans une direction fondamentalement tangentielle.

10. Embout d'oreille (80) selon l'une quelconque des revendications précédentes, dans lequel le second bras (86) est plus long que le premier bras (84) et dans lequel le second bras (86) fait saillie d'une surface circonférentielle (104) du corps principal (82) dans une direction fondamentalement radiale.

11. Embout d'oreille (80) selon l'une quelconque des revendications précédentes, dans lequel, à l'état non sollicité, le premier bras (84) et le second bras (86) sont incurvés, avec la même direction de courbure, et dans lequel un rayon de courbure du premier bras (84) est plus grand qu'un rayon de courbure du second bras (86).

12. Embout d'oreille (80) selon l'une quelconque des revendications précédentes, dans lequel l'embout d'oreille (80) est une pièce de moulage par injection à composants multiples comportant au moins deux composants ayant différentes propriétés matérielles.

13. Embout d'oreille (80) selon l'une quelconque des revendications précédentes, dans lequel, sur le corps principal (82), il est prévu une interface de liaison (110) qui est agencée pour être engagée par une interface d'accouplement (160) d'une pièce de fixation (152).

14. Référence de position (150) pour un système de traitement de cheveux automatisé (10), la référence de position (150) comprenant une unité de référence (154) et un embout d'oreille (80) selon l'une quelconque des revendications précédentes montable dans l'oreille, dans laquelle l'unité de référence (154) est fixée à l'embout d'oreille (80).

15. Système de traitement de cheveux automatisé (10), en particulier système de coupe de cheveux, le système comprenant un appareil de traitement de cheveux manuel portable (20), une unité de traitement de cheveux (24) agencée sur l'appareil (20) et un agencement de surveillance de position (30) comprenant au moins une référence de position (150) selon la revendication 14, dans lequel au moins un élément de l'appareil de traitement de cheveux (20) peut être commandé en fonction d'au moins l'une d'une orientation réelle et d'une position réelle de l'appareil de traitement de cheveux (20) par rapport à la tête d'un sujet de traitement.
